# EUROPEAN PATENT SPECIFICATION

(11) **EP 1 699 750 B1**
(45) Date of publication and mention of the grant of the patent: **06.11.2013**
(21) Application number: 04804882.1
(22) Date of filing: 16.12.2004
(51) Int. Cl.: C07C 67/03, C07C 69/533

(54) **A PROCESS FOR THE PREPARATION OF AN ALKYL ALKENOATE**
VERFAHREN ZUR HERSTELLUNG EINES ALKYLALKENOATES
PROCÉDÉ DE PRÉPARATION D'ALCÈNOATE D'ALKYLE

(30) Priority: 19.12.2003 EP 03258057
(43) Date of publication of application: 13.09.2006
(73) Proprietor: Shell Internationale Research Maatschappij B.V., 2596 HR Den Haag (NL)
(72) Inventor: HAAN, Rene Johan, NL-1031 CM Amsterdam (NL); LANGE, Jean-Paul, NL-1031 CM Amsterdam (NL)
(74) Representative: Matthezing, Robert Maarten
(86) International application number: PCT/EP2004/053536
(87) International publication number: WO 2005/058793

(56) References cited:
- US-A- 4 740 613
- US-A- 4 777 285
- US-A- 5 144 061
- M. SAKUTH, D. REUSCH, R. JANOWSKY: "Ullmann's Encyclopedia of Industrial Chemistry : Reactive Distillation - 1. Indroduction" [Online] 15 June 2000 (2000-06-15), WILEY-VCH VERLAG GMBH & CO KGAA , XP002282302 Retrieved from the Internet: URL:http://www.mrw.interscience.wiley.com/ ueic/articles/c22_c01/sect1-fs.html> the whole document

## Description

### Field of the Invention

The present invention relates to a process for the preparation of an alkyl alkenoate.

### Background of the Invention

Pentenoic acid and its esters, can be converted into caprolactam or adipic acid, which are both important nylon precursors. It is known to prepare pentenoic acid or its esters via carbonylation of butadiene. In EP 738 701 for example, a process for the preparation of 3-pentenoic acid is disclosed. Butadiene is hydroxycarbonylated by reacting it with carbon monoxide and water in the presence of an iridium catalyst and an iodide or bromide promoter. In EP 728 733 and EP 728 732, a carbonylation process for the preparation of methyl pentenoate is disclosed, wherein butadiene is reacted with carbon monoxide and methanol in the presence of a carbonylation catalyst system comprising palladium, a carboxylic acid and a phosphine ligand.

Alternatively, alkyl pentenoates can be prepared by contacting an alkoxy-butene and carbon monoxide in the presence of a carbonylation catalyst. In WO 96/29300, for example, a process for the preparation of methyl pentenoate is described wherein a mixture of 3-methoxy-1-butene and 1-methoxy-2-butene is carbonylated with carbon monoxide in the presence of a catalyst. In US 6,175,036, a similar process is disclosed.

In EP 395 038, a process is disclosed for the preparation of adipic acid, wherein a lactone is reacted with carbon monoxide and water in the presence of a homogeneous rhodium catalyst and an iodide or bromide promoter.

The above-mentioned carbonylation processes have several disadvantages. Carbonylation requires catalysts comprising expensive noble metals and expensive ligands. Since the catalysts are homogeneous catalysts, it is difficult to recover all of the noble metals and the ligands from the reaction products. Moreover, the processes require the use of carbon monoxide, which is a toxic compound.

An alternative route for the preparation of alkyl pentenoates and other alkyl alkenoates is the transesterification reaction of a lactone with an alcohol, in particular the reaction of gamma valerolactone with methanol to form methyl pentenoate.

In US 5,144,061 for example, it is disclosed that a gas phase reaction of gamma valerolactone (5-methyl-butyrolactone) with methanol (lactone-to-alcohol molar ratio is 1:3) in the presence of an acidic zeolitic catalyst results in 13-72 % conversion to methyl pentenoate. The selectivity towards 4-pentenoate and 3-pentenoate is higher than that towards 2-pentenoate.

In US 4,740,613 is also disclosed the gas phase reaction of gamma valerolactone with methanol. Lactone-to-alcohol molar ratios of 1:1 and 1:4 are exemplified. A lactone-to-alcohol molar ratio from 1:0.5 to 1:10 is mentioned. Also in US 4,740,613 it is mentioned that it is possible to carry out the reaction in the liquid phase.

In US 4,777,285 the preparation of alkyl pentenoates from reacting 6-membered or 7-membered lactones with an alcohol is disclosed. Gas phase reactions with a lactone-to-methanol ratio of 1:1 and 1:3.6 are exemplified. A lactone-to-alcohol ratio from 1:0.5 to 1:10 is mentioned.

It is mentioned in US 5,144,061, US 4,740,613, and US 4,777,285 that it is possible to carry out the reaction in the liquid phase.

A disadvantage of the processes as disclosed in US 5,144,061, US 4,740,613, and US 4,777,285 is that under the reaction conditions applied, part of the alcohol will be converted into a di-ether and heavier further products and is thus not available for the desired reaction.

### Summary of the Invention

It has now been found that alkyl alkenoates can be prepared with a high conversion and with hardly any di-ether formation from a lactone as starting material by reacting the lactone and an alkyl alcohol with each other under distillative transesterification conditions in the presence of a strong acid catalyst.

Accordingly, the present invention relates to a process for the preparation of an alkyl alkenoate, wherein a lactone of the general molecular formula 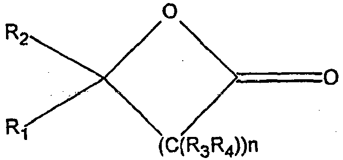 wherein n is 1, 2 or 3, R₁ is a C₁-C₄ alkyl group, and R₂, R₃ and R₄ are, independently, a H atom or a C₁-C₄ alkyl group,
is reacted with a C₁-C₄ alkyl alcohol in a liquid phase in the presence of a strong acid catalyst at transesterification conditions to form the alkyl alkenoate, wherein alkyl alkenoate and alcohol are continuously removed from the liquid phase by distillation.

By distilling off the alcohol from the liquid phase in which the reaction takes place, the lactone-to-alcohol ratio in the liquid reaction phase is much higher than in the gas or liquid phase processes disclosed in the above-mentioned prior art. Despite the low alcohol concentration in the liquid phase, a surprisingly high yield of alkyl alkenoates is achieved in the process according to the invention.

### Brief Description of the Drawings

Two embodiments of the invention are described in detail and by way of example with reference to drawings 1 and 2.

Figure 1 schematically depicts a flow diagram of a first embodiment, using a distillation column with an homogeneous catalyst.

Figure 2 schematically depicts a flow diagram of a second embodiment, using a distillation column with an heterogeneous catalyst.

### Detailed Description of the Invention

In the process according to the invention, a lactone and an alcohol are reacted with each other in a liquid phase to form an alkyl alkenoate in the presence of a strong acid catalyst under reactive distillation conditions.

The lactone may be any 4-, 5-, or 6-membered ring lactone, wherein the ring carbon atom that is next to the ring-closing oxygen atom is substituted with a C₁-C₄ alkyl group. The other ring carbon atoms may be unsubstituted or substituted with C₁-C₄ alkyl groups. The lactone is of the general molecular formula 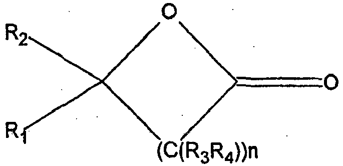 wherein n is 1, 2 or 3, R₁ is a C1-C4 alkyl group and R₂, R₃ and R₄ are, independently, a H atom or a C₁-C₄ alkyl group.

The ring carbon atom that is next to the ring-closing oxygen atom is preferably substituted with a methyl or ethyl group, more preferably with a methyl group. Thus, R₁ is preferably methyl or ethyl, more preferably methyl. R₂ is preferably a hydrogen atom.

The other ring carbon atoms are preferably unsubstituted. Therefore, both R₃ and R₄ are preferably a hydrogen atom for each carbon atom. If the other ring carbon atoms are substituted and n is 2 or 3, the R₃ or R₄ groups may differ for each carbon atom.

The lactone is preferably a 5-membered ring lactone. Thus, n is preferably 2.

Examples of suitable lactones are gamma valerolactone, delta caprolactone or gamma caprolactone. A particularly suitable lactone is gamma valerolactone. If the lactone is gamma valerolactone, the alkyl alkenoate that is formed is alkyl pentenoate. Under the acid conditions of the process of the invention, a mixture of 2-, 3-, and 4-pentenoate will be formed with the 2- and 3-pentenoate both in the cis and the trans configuration.

In the process according to the invention, a lactone and an alcohol in a liquid phase are reacted with each other at transesterification conditions in the presence of a strong acid catalyst. Alkyl alkenoate is formed by the transesterification reaction and the alkyl alkenoate and alcohol are continuously removed from the liquid reaction mixture by distillation. Although gaseous alcohol is present in the process according to the invention, the reaction takes place between lactone and alcohol in the liquid phase.

Since alcohol is continuously removed from the liquid reaction phase, the lactone-to-alcohol ratio in the liquid phase is high. Preferably, the lactone-to-alcohol molar ratio in the liquid phase is at least 3, more preferably at least 5, even more preferably at least 10.

It will be appreciated that the reaction is carried out at such a temperature that the alkyl pentenoate can be distilled from the liquid phase. Therefore, the temperature is above the boiling temperate of the lowest-boiling azeotrope containing the alkyl pentenoate and below the boiling temperature of the lactone. The exact temperature will thus depend on the reactants used and on the operating pressure. Typically, the temperature will be in the range of from 100 to 300 °C, preferably in the range of from 150 to 250 °C.

The process may be operated at any suitable pressure. It is an advantage of the process according to the invention that no high pressure conditions are needed. Preferably, the pressure is in the range of from 0.01 to 10 bar (absolute), more preferably of from 0.1 to 5 bar (absolute), even more preferably the pressure is ambient pressure.

It will be appreciated that a difference in boiling temperature between the lactone and the alkyl alkenoate is needed in the process according to the invention. In order to have such a difference in boiling point, the alcohol with which the lactone is reacted is a C₁-C₄ alkyl alcohol, preferably methanol or ethanol, more preferably methanol.

The process according to the invention may be catalysed by an homogeneous or an heterogeneous strongly acidic catalyst. Thus, the catalyst may be a liquid or a solid strong acid. Preferred liquid strong acids are sulphuric acid or p-toluene sulphonic acid (pTSA). Examples of suitable strongly acidic solids are ion-exchange resins and strongly acidic mixed oxides such as acidic crystalline silica-alumina (zeolites). Preferred strongly acidic solid catalysts are ion-exchange resins, acidic ZSM-5 zeolite and acidic beta zeolite.

The process according to the invention may be operated as a batch, semi-batch or continuous process. Preferably, the process is operated as a continuous process wherein lactone and alcohol are continuously supplied to the reaction zone and alkyl alkenoate, alcohol, and water are continuously removed from the reaction zone by distillation. More preferably, the continuous process is performed in a distillation column.

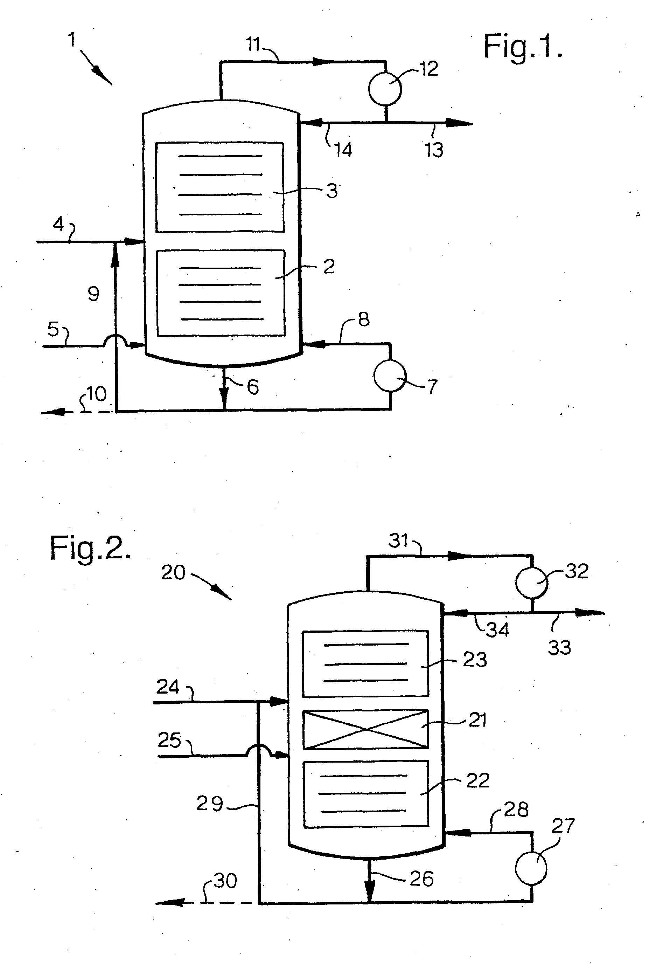

It is known in the art how to design a distillation column for reactive distillation purposes. Reference is for example made to G. Ertl, H. Knözinger, J. Weitkamp (Eds), Handbook of Heterogeneous Catalysis, Volume 3, 1997, VCH, pp 1479-1487. It will be appreciated that a design for a reactive distillation column for an homogeneous catalytic reaction is different from one for an heterogeneous catalytic reaction. For illustrative purposes, two embodiments of the process of the invention are shown in Figures 1 and 2, one for homogeneous catalysis (Figure 1) and one for heterogeneous catalysis (Figure 2).

If the process is performed in a distillation column and the catalyst is a solid catalyst (heterogeneous catalysis), the distillation column comprises a reaction zone containing the solid catalyst. This reaction zone has both a reaction and a distillation function. Suitable solid catalyst structures for such a double function reaction zone are known in the art, for example from US 4,443,559. The distillation column preferably comprises additional distillation zones above and/or below the reaction zone. These additional distillation zones each comprise at least one theoretical fractionation stage in the form of conventional distillation trays or inert packing.

The homogeneous catalyst is preferably present in a concentration in the range of from 0.01 to 20% by weight based on the weight of lactone in the reaction zone, more preferably of from 0.1 to 10% by weight. If the catalyst is an heterogeneous catalyst and the process is performed as a batch or semi-batch process, the preferred catalyst concentrations are as described above for the homogeneous catalyst.

It will be appreciated that in the case of an heterogeneous catalyst in a reactive distillation column, it is more appropriate to express the catalyst concentration in terms of a weight hourly velocity of the lactone. Preferably, the lactone weight hourly velocity is in the range of from 0.1 to 10 kg lactone per kg catalyst per hour, more preferably of from 0.5 to 5 kg/kg/h.

### Detailed description of the drawings

Figure 1 shows a distillation column 1, which has a stripping section 2 and a rectifying section 3, each section comprising at least one theoretical fractionation stage. Before starting normal operation of the column, a strong liquid acid catalyst, e.g. sulphuric acid, is introduced into column 1. During normal operation of distillation column 1, gamma valerolactone, optionally with some acid catalyst, is continuously introduced in column 1 via line 4 at a point between stripping section 2 and rectifying section 3. Methanol is continuously introduced into column 1 below stripping section 2, via line 5. The methanol may be introduced in column 1 as a vapour or may be vaporised upon entering column 1. A liquid bottoms stream comprising gamma valerolactone and the acid catalyst is withdrawn from distillation column 1 via line 6. Part of the bottoms stream is vaporised in reboiler 7 and re-introduced in column 1 via line 8. Part of the liquid bottoms stream is recycled to feed line 4 via line 9 and re-introduced in column 1 with the fresh lactone. A small part of the bottoms stream may be purged via line 10. A gaseous overhead stream comprising methyl pentenoate, water and methanol is withdrawn from column 1 via line 11 and condensed in condenser 12. Part of the condensed stream is withdrawn as product stream via line 13. The remainder is reintroduced in column 1 just above rectifying section 3, via line 14.

It will be appreciated that the reaction between gamma valerolactone and methanol takes place in that part of the column where the acid catalyst and the reactants are present, i.e. the part of the column between the methanol inlet and the inlet of the partly condensed bottoms stream. The height of the inlet of the partly condensed bottoms stream thus determines the length of the reaction zone.

In Figure 2 is shown a distillation column 20, which has a reaction zone 21 containing the solid acidic catalyst, a stripping section 22 below the catalyst bed and a rectifying section 23 above the catalyst bed. The stripping and rectifying sections 22, 23 and the reaction zone 21 each comprise at least one theoretical fractionation stage. Gamma valerolactone is introduced into column 20 above reaction zone 21 via line 24 and methanol is introduced into column 20 below reaction zone 21 via line 25. A liquid bottoms stream comprising gamma valerolactone is withdrawn from distillation column 20 via line 26. Part of the bottoms stream is vaporised in reboiler 27 and re-introduced in column 20 via line 28. Part of the liquid bottoms stream is recycled to feed line 24 via line 29 and re-introduced in column 20 with the fresh lactone. A small part of the bottoms stream may be purged via line 30. A gaseous overhead stream comprising methyl pentenoate, water and methanol is withdrawn from column 20 via line 31 and condensed in condenser 32. Part of the condensed stream is withdrawn as product stream via line 32. The remainder is reintroduced in column 20 just above rectifying section 23, via line 34.

In the embodiments of both Figures 1 and 2, methyl pentenoate and/or methanol may be separated from the product stream comprising methyl pentenoate, methanol and water by means known in the art (not shown), such as fractionation, extraction, liquid/liquid separation, or a combination thereof. The separated methanol may then be recycled to line 5 or line 25.

### Examples

The invention is now illustrated by means of the following non-limitative examples.

### EXAMPLES 1-14

A 250 ml three-neck glass flask was provided with a rectification column with a length of 20 cm, which was filled with metal barrel seeds and was connected to a water cooler and collection flask. The three-neck flask was loaded with 80 g lactone and acid catalyst. The flask was heated to 190 °C and alcohol was continuously fed to the flask. The volatile compounds, i.e. alkyl pentenoate, alcohol, and water, were stripped from the reaction mixture by a flow of nitrogen of 0.6 litre per hour. The distillate product was collected and analysed by GLC. Different experiments were carried out using different catalysts, alcohols and lactones. All experiments were performed at ambient pressure.

In EXAMPLE 5, 60 g gamma valerolactone, 30 g methanol, and 2 g pTSA were loaded in a 300 ml steel autoclave, purged with nitrogen and pressurised to 1 bar (absolute) with nitrogen. The autoclave was heated to 200 °C under autogeneous pressure. After 26 hours, the autoclave was cooled down and the liquid product analysed by GLC. In this experiment, methanol and methyl pentenoate were thus not distilled from the liquid phase.

The Table below shows the reactants, catalyst, alcohol feed rate, duration and product yield for each experiment. Examples 1-4, 6-9, and 13 are examples according to the invention; examples 5, 10-12, and 14 are comparative examples. In the Examples 1-4 and 6-14, only a negligible amount (<< 1% of the amount of alcohol supplied to the process) of di-ether (di-ethyl ether in example 13; di-methyl ether in the other examples) was formed.

The product yield as shown in the Table is the yield calculated from the amount of pentenoate found in the distillate. The amount of pentenoate that is formed might be somewhat higher, since some pentenoate might have remained in the reaction flask.

The liquid reaction mixture obtained after the experiment in EXAMPLE 5 (no distillation) contained 50 g unconverted gamma valerolactone, 2.5 g methyl pentenoate, 2.4 g of heavy products, methanol and a significant amount of di-methyl ether.

**Table Results of examples 1-14**

| example | lactone¹ | alcohol² | catalyst³ | catalyst conc. (mg/g lactone) | feed rate alcohol (mg/g lactone.h) | time (h) | mole% product |
|---|---|---|---|---|---|---|---|
| homogeneous strong acid catalysts | | | | | | | |
| 1 | gVL | MeOH | H₂SO4 | 20 | 76 | 6.5 | 56 |
| 2 | gVL | MeOH | H₂SO4 | 16 | 62 | 6 | 33 |
| 3 | gVL | MeOH | pTSA | 62 | 73 | 6 | 37 |
| 4 | gVL | MeOH | pTSA | 31 | 74 | 6 | 23 |
| | | | | | | 25 | 91 |

| 5 (comparison) no distillation | gVL | MeOH | pTSA | 33 | 0 | 26 | 3.7 |
|---|---|---|---|---|---|---|---|
| heterogeneous strong acid catalysts | | | | | | | |
| 6 | gVL | MeOH | H-β 200 | 53 | 77 | 6 | 25 |
| 7 | gVL | MeOH | H-β 22 | 56 | 77 | 6 | 26 |
| 8 | gVL | MeOH | H-ZSM-5 | 42 | 73 | 6.2 | 29 |
| 9 | gVL | MeOH | Nafion NR-50 | 29 | 74 | 5.3 | 34 |

| heterogeneous medium and weak acid catalysts | | | | | | | |
|---|---|---|---|---|---|---|---|
| 10 (comparison) | gVL | MeOH | ASA | 28 | 82 | 5.5 | 1.7 |
| 11 (comparison) | gVL | MeOH | γ-Al₂O₃ | 50 | 74 | 6.5 | < 0.1 |
| 12 (comparison) | gVL | MeOH | La/Al₂O₃ | 66 | 95 | 5.5 | < 0.1 |

| Different alcohol | | | | | | | |
|---|---|---|---|---|---|---|---|
| 13 | gVL | EtOH | pTSA | 32 | 69 | 27 | 22.5 |

| Different lactone | | | | | | | |
|---|---|---|---|---|---|---|---|
| 14 (comparison) | gBL | MeOH | pTSA | 40 | 32 | 5.7 | - |

| | | | | | | | |
|---|---|---|---|---|---|---|---|
| ¹ gVL: gamma valerolactone; gBL: gamma butyrolactone. ² MeOH: methanol; EtOH: ethanol. ³ H-β 22, H-β 200: acidic beta zeolite with a silica-to-alumina molar ratio of 22 and 200, respectively; H-ZSM-5: acidic ZSM-5 zeolite; Nafion NR-50: commercially available ion-exchange resin (ex. Dupont); ASA: amorphous silica-alumina; La/Al₂O₃: 16% by weight La on alumina. | | | | | | | |

## Claims

1. A process for the preparation of an alkyl alkenoate, wherein a lactone of the general molecular formula 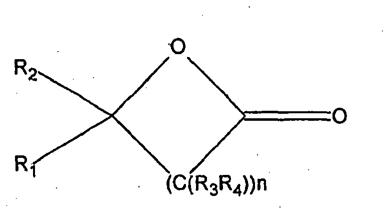 wherein n is 1, 2 or 3, R₁ is a C₁-C₄ alkyl group, and R₂, R₃ and R₄ are, independently, a H atom or a C₁-C₄ alkyl group,
is reacted with a C₁-C₄ alkyl alcohol in a liquid phase in the presence of a strong acid catalyst at transesterification conditions to form the alkyl alkenoate, wherein alkyl alkenoate and alcohol are continuously removed from the liquid phase by distillation.

2. A process according to claim 1, wherein R₁ is an ethyl or a methyl group, preferably a methyl group.

3. A process according to claim 1 or 2, wherein R₂ is a hydrogen atom.

4. A process according to any one of the preceding claims, wherein both R₃ and R₄ are a hydrogen atom.

5. A process according to any one of the preceding claims, wherein n is 2.

6. A process according to any one of the preceding claims, wherein the lactone is gamma valerolactone and the alkyl alkenoate is alkyl pentenoate.

7. A process according to any one of the preceding claims, wherein the lactone-to-alcohol molar ratio in the liquid phase is at least 3, preferably at least 5, more preferably at least 10.

8. A process according to any one of the preceding claims, wherein the reaction is carried out at a temperature in the range of from 100 to 300 °C, preferably of from 150 to 250 °C.

9. A process according to any one of the preceding claims, wherein the pressure in the reaction zone is in the range of from 0.01 to 10 bar (absolute), preferably of from 0.1 to 5 bar (absolute), more preferably ambient pressure.

10. A process according to any one of the preceding claims, wherein the alkyl alcohol is methanol or ethanol, preferably methanol.

11. A process according to any one of the preceding claims, wherein the catalyst is a strong liquid acid, preferably sulphuric acid or p-toluene sulphonic acid.

12. A process according to any one of claims 1 to 9, wherein the catalyst is a strongly acidic solid, preferably an ion-exchange resin or acidic ZSM-5 or beta zeolite.

## Patentansprüche

1. Verfahren zur Herstellung eines Alkylalkenoats, wobei ein Lakton der allgemeinen Strukturformel 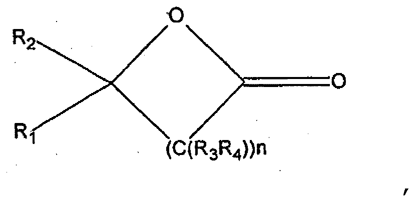 wobei n 1, 2 oder 3 beträgt, R₁ eine C₁-C₄-Alkylgruppe bedeutet und R₂, R₃ und R₄ unabhängig ein H-Atom oder eine C₁-C₄-Alkylgruppe darstellen,
mit einem C₁-C₄-Alkylalkohol in einer flüssigen Phase in Gegenwart eines starken Säurekatalysators bei Umesterungsbedingungen umgesetzt wird, um das Alkylalkenoat auszubilden, wobei Alkylalkenoat und Alkohol kontinuierlich aus der flüssigen Phase durch Destillation entfernt werden.

2. Verfahren nach Anspruch 1, wobei R₁ eine Ethyl- oder eine Methylgruppe, vorzugsweise eine Methylgruppe, ist.

3. Verfahren nach Anspruch 1 oder 2, wobei R₂ ein Wasserstoffatom ist.

4. Verfahren nach einem der vorstehenden Anspruche, wobei sowohl R₃ als auch R₄ ein Wasserstoffatom ist.

5. Verfahren nach einem der vorstehenden Ansprüche, wobei n 2 beträgt.

6. Verfahren nach einem der vorstehenden Ansprüche, wobei das Lakton Gamma-Valerolakton ist und das Alkylalkenoat Alkylpentenoat ist.

7. Verfahren nach einem der vorstehenden Ansprüche, wobei das Molverhältnis von Lakton zu Alkohol in der flüssigen Phase mindestens 3, vorzugsweise mindestens 5, stärker bevorzugt mindestens 10 beträgt.

8. Verfahren nach einem der vorstehenden Ansprüche, wobei die Reaktion bei einer Temperatur im Bereich von 100 bis 300 °C, vorzugsweise von 150 bis 250 °C ausgeführt wird.

9. Verfahren nach einem der vorstehenden Ansprüche, wobei der Druck in der Reaktionszone im Bereich von 0,01 bis 10 bar (absolut), vorzugsweise von 0,1 bis 5 bar (absolut), stärker bevorzugt Umgebungsdruck beträgt.

10. Verfahren nach einem der vorstehenden Ansprüche, wobei der Alkylalkohol Methanol oder Ethanol, vorzugsweise Methanol ist.

11. Verfahren nach einem der vorstehenden Ansprüche, wobei der Katalysator eine starke flüssige Säure, vorzugsweise Schwefelsäure oder p-Toluolsulfonsäure ist.

12. Verfahren nach einem der Ansprüche 1 bis 9, wobei der Katalysator ein stark saurer Feststoff, vorzugsweise ein Ionenaustauscherharz oder saures ZSM-5 oder beta-Zeolith ist.

## Revendications

1. Procédé de préparation d'un alkénoate d'alkyle, **caractérisé en ce qu'** une lactone de formule moléculaire générale 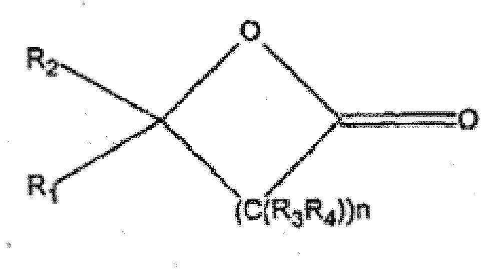 **caractérisé en ce que** n est 1, 2 ou 3, R1 est un groupe alkyle en C1-C4, et R2, R3 et R4 sont, indépendant, un atome de H ou un groupe alkyle C1-C4,
est mise à réagir avec un alcool d'alkyle C1-C4 en phase liquide en présence d'un catalyseur acide fort dans des conditions de transestérification pour former l'alkénoate d'alkyle, où l'alkénoate d'alkyle et l'alcool sont éliminés en continu de la phase liquide par distillation.

2. Procédé selon la revendication 1, **caractérisé en ce que** R1 est un groupe éthyle ou méthyle, de préférence un groupe méthyle.

3. Procédé selon la revendication 1 ou 2, **caractérisé en ce que** R2 est un atome d'hydrogène.

4. Procédé selon l'une quelconque des revendications précédentes, **caractérisé en ce que** R3 et R4 sont un atome d'hydrogène.

5. Procédé selon l'une quelconque des revendications précédentes, **caractérisé en ce que** n est 2.

6. Procédé selon l'une quelconque des revendications précédentes, **caractérisé en ce que** la lactone est la gamma-valérolactone et l'alkénoate d'alkyle est le penténoate d'alkyle.

7. Procédé selon l'une quelconque des revendications précédentes, **caractérisé en ce que** le rapport molaire lactone-alcool dans la phase liquide est au moins 3, de préférence au moins 5, ou mieux au moins 10.

8. Procédé selon l'une quelconque des revendications précédentes, **caractérisé en ce que** la réaction est effectuée à une température de l'ordre de 100 à 300 °C, de préférence de 150 à 250 °C.

9. Procédé selon l'une quelconque des revendications précédentes, **caractérisé en ce que** la pression dans la zone de réaction est de l'ordre de 0,01 à 10 bar (pression absolue), de préférence 0,1 à 5 bars (pression absolue), ou, mieux, la pression ambiante.

10. Procédé selon l'une quelconque des revendications précédentes, **caractérisé en ce que** l'alcool d'alkyle est le méthanol ou l'éthanol, de préférence le méthanol.

11. Procédé selon l'une quelconque des revendications précédentes, **caractérisé en ce que** le catalyseur est un acide liquide fort, de préférence l'acide sulfurique ou l'acide p-toluène sulfonique.

12. Procédé selon l'une quelconque des revendications 1 à 9, **caractérisé en ce que** le catalyseur est un solide fortement acide, de préférence une résine à échange ionique ou une zéolite ZSM-5 acide ou une zéolite bêta.
